# EUROPEAN PATENT SPECIFICATION

(11) **EP 0 677 045 B1**
(45) Date of publication and mention of the grant of the patent: **10.02.1999**
(21) Application number: 94903426.8
(22) Date of filing: 03.12.1993
(51) Int. Cl.: C07D 277/80, C07D 277/78, C07D 213/71, C07C 333/32, C07C 321/22, C07D 239/38

(54) **CATALYTIC OXIDATION PROCESS OF THIOLS**
VERFAHREN ZUR KATALYTISCHEN OXYDATION VON THIOLEN
PROCEDE D'OXYDATION CATALYTIQUE DE THIOLS

(30) Priority: 30.12.1992 US 998713
(43) Date of publication of application: 18.10.1995
(73) Proprietor: MONSANTO COMPANY, St. Louis, Missouri 63167 (US)
(72) Inventor: COMEYNE, Denis, Oscar, Longmeadow, MA 01106 (US); MEERBERGEN, Eric, Jean, Akron, OH 44333 (US); RILEY, Dennis, Patrick, Ballwin, MO 63011 (US); SIKORA, David, John, Hudson, OH 44236 (US); STRACKX, Gilbert, Francois, Jozef, 26 Berchem B-Antwerp (BE); YOUNG, Mary, Jennifer, Torres, League City, TX 77573 (US)
(74) Representative: Colens, Alain
(86) International application number: US9311740
(87) International publication number: WO9415927

(56) References cited:
- EP-A- 0 131 776
- FR-A- 2 521 559
- GB-A- 2 062 631
- US-A- 3 654 297
- US-A- 4 182 873
- US-A- 4 461 897

## Description

This invention relates to a process for the catalytic oxidation of thiols, using oxygen, in which the catalyst is carbon.

### BACKGROUND OF THE INVENTION

Processes for the oxidation of thiols or thiol salts are known in the prior art. Some of these processes use halogen or hypohalite oxidants, which are sufficiently powerful so they do not require catalysts. Others, which use oxygen or a mixture of oxygen and an inert gas (such as air), require a catalyst in order to achieve practical reaction rates. This invention is concerned with the latter type of reactions.

A number of processes have been disclosed for oxidizing a thiol such as 2-mercaptobenzothiazole with oxygen to produce benzothiazyl disulfide. U. S. Patent 3,654,297, in the name of Goulandris, shows such a process, using a catalyst which is a salt of cobalt phthalocyanine.

Other processes are known in which a thiol is oxidized with oxygen in the presence of a primary or secondary amine, producing a sulfenamide. U. S. Patent 3,737,431, to Campbell et al, shows oxidation of 2-mercaptobenzothiazole in the presence of a primary or secondary amine, using oxygen and a metal phthalocyanine catalyst, to produce the corresponding sulfenamides. Cobb et al in U. S. Patent 4,461,897 disclose a similar reaction, in which the metal phthalocyanine catalyst is supported on a water-insoluble, solid, adsorbent support such as clay or activated carbon.

Still other known processes show oxidation of carbon disulfide together with a secondary amine, using oxygen and a phthalocyanine catalyst, to form thiuram disulfides. (U. S. Patents 3,116,328 to Cox et al and 3,116,329 to Hayes et al).

Yet another process, in U. S. Patent 5,124,450 to Bergfeld et al, shows the preparation of 2-amino di- and trithiothiazoles by oxidizing a mixture of 2-mercaptothiazoles, secondary cyclic amines and sulfur, using oxygen and a catalyst which is metallic copper, a copper compound or a cerium compound.

All of the above processes for the catalytic oxidation of thiols have one common drawback: they involve a metal component. Such metal components not only make the catalyst more expensive, initially, but raise important environmental concerns. For example, effluent streams can be contaminated by the metals, and recovery of the catalyst can be prohibitively expensive.

### SUMMARY OF THE INVENTION

It has now been discovered that thiols can be oxidized with oxygen, in the presence of selected amine, through the use of a carbon catalyst, which contains no metal complex of porphyrazine or of a derivative of porphyrazine. The process is performed in a liquid reaction medium comprising protic or aprotic solvents at a temperature of from at least 25°C to 150°C.

Suitable thiols which are oxidized in the process of the invention are thiols of nitrogen-containing heterocycles.

Examples of such thiols include 2-mercaptobenzothiazole, 2-pyridinethiol, 2-pyrimidinethiol, 4-pyrimidinethiol, 2-pyrazinethiol, 3-pyridazinethiol, 2-mercaptobenzimidazole, 2-quinolinethiol and 2-lepidinethiol.

Oxygen can be used in the process of the invention, either by itself, or in combination with another, inert gas, such as in air.

The primary and secondary amines useful in the process of the invention include isopropylamine, t-butylamine, cyclohexylamine, diisopropylamine, and morpholine. The use of a mixture of two or more amines can result in a mixture of sulfenamides in the product.

Optionally, the process of the invention may also include sulfur in its starting materials, in addition to an amine such as morpholine, and a thiol of a nitrogen-containing heterocycle, such as 2-mercaptobenzothiazole. In this specific instance, the product of the process is a 2-aminodithiothiazole or a 2-aminotrithiothiazole, depending on the amount of sulfur charged.

The catalyst used in the process of the invention is carbon. Preferably, it is activated carbon, which can be charcoal which has been heat-treated or chemically treated or both to form a highly porous particle structure of increased adsorbent capacity. The carbon source itself can be wood, coal, petroleum, natural gas, peat, nut shells or bones.

Especially preferred is activated carbon which has been treated so as to remove oxides from its surface. Particularly effective for this purpose is the process disclosed and claimed in U. S. Patent 4,624,937 in the name of S. K. Chou, the disclosure of which is herein incorporated by reference. Briefly summarized, the process involves oxidizing and pyrolyzing the carbon, either in two successive steps or in a single step. Oxidants include the combination of ammonia (NH₃) and an oxygen-containing gas such as H₂O, NOₓ, CO₂, or SO₂. Pyrolysis temperatures typically range from 800° to 1200°C.

### DETAILED DESCRIPTION OF THE INVENTION

The process of the invention is usually performed at temperatures of at least from 25° to 150°C and a preferred range is 30° to 90°C.

Oxygen or air is usually supplied to the reaction zone under pressure, which should be sufficient to drive the reaction, and can be as high as is practical to handle. Preferably, the pressure will be between 0.1 and 1.0 MPa. When air is used, pressures will typically be higher.

At the preferred pressure and temperatures, the reaction will proceed to completion in a reasonable time, that is, from 0.1 to 10 hours. Usually, higher temperatures and pressures will produce shorter reaction times.

The reaction is performed in a liquid reaction medium, comprising protic or non-protic solvents. In those reactions wherein an amine is present, an excess of the amine can serve as a liquid reaction medium. Also, when the process of the invention is employed to produce a sulfenamide, a small amount of water may be charged, as is disclosed in Cobb et al U. S. Patent 4,461,897.

The carbon catalyst can be used in an amount of from 1% to 75% by weight, based on the thiol. The use of higher amounts can be impractical; lower amounts give very slow reaction rates.

A more complete understanding of the invention may be obtained by reference to the following examples, in which all temperatures are in degrees celsius unless otherwise indicated.

### EXAMPLE 1

This example describes the preparation of N-tert-butyl-2-benzothiazole sulfenamide (TBBS). A solution of 5.35 g (0.03 mole) of 2-mercaptobenzothiazole (MBT), 73.1 g of tert-butylamine (1.0 mole), and 10 g of water was placed in a 300 mL Autoclave Engineers autoclave equipped with a temperature control system, mass flow controller, stirrer, and an oxidation-reduction potential (ORP) probe and recorder. To this was added 1.2 g of an activated carbon catalyst which had been treated to remove surface oxides according to the method of U. S. Patent 4,624,937. The autoclave was capped and heated to 50°C. Oxygen flow was started at 37 mL/min to a gauge pressure of 0.12 MPa and the reaction temperature increased to 71°C within 3 min. Oxygen consumption and a change in the ORP of the reaction mixture was detected. The reaction was stopped after 48 min reaction time, by which time a plateau in the ORP measurement was noted. The mixture was cooled to 30°C and the autoclave was opened. After the catalyst was allowed to settle, HPLC analysis of the crude liquid showed a selective conversion to TBBS (98.6%). No MBT could be detected and there was no indication of significant amounts of by-products. TBBS in the crude liquid was precipitated by the addition of an excess amount of water (2:1 v/v water:amine). Filtration, washing with cold water, and drying of the precipitate at 105°C for 2 hours yielded TBBS with very high purity of 99.5% as measured by HPLC and amine titration.

### EXAMPLE 2

The procedure was as in Example 1, but with the use of only 3.3 g of water and in the absence of carbon catalyst, or any catalyst. No significant reaction was observed in terms of oxygen consumption or ORP change within 30 minutes. HPLC analysis of the crude liquid showed essentially no conversion of MBT to TBBS.

### EXAMPLE 3

The procedure was as in Example 1, but with the exclusion of water from the reaction mixture. Because MBT was not completely dissolved in tert-butylamine, ORP monitoring of the reaction was not possible. A significant temperature increase was observed within a few minutes after the start of oxygen flow. After 30 minutes of reaction, HPLC analysis of the crude reaction mixture indicated the formation of >96% of TBBS with <0.1% MBT and no significant amounts of side products. This result indicates that the oxidation of MBT to TBBS using the activated carbon catalyst of Example 1 can be performed in an initially anhydrous medium.

### EXAMPLE 4

This example illustrates the use of another activated carbon catalyst. The procedure was as in Example 1, but with the use of 1.2 g Norit SX3 SM activated carbon catalyst. The reaction mixture was heated to 40°C before oxygen flow was started. ORP monitoring of the reaction indicated good conversion of MBT within a 30-minute reaction time. The crude liquid by HPLC analysis was composed of 1.05% MBT, 90.5% TBBS, 0.26% MBTS and 2.65% benzothiazole.

### EXAMPLE 5

This example describes the preparation of TBBS using air as the oxidant. The procedure was as in Example 1, but to compensate for the lower oxygen content of air, the reaction was run at 0.35 MPa gauge pressure between 30-50°C. Both the ORP measurement and the rate of oxygen consumption indicated a slower reaction. The reaction was stopped after 2 hours and the crude liquid, as determined by HPLC analysis was composed of 18.0% MBT, 79.3% TBBS and 0.2% MBTS.

### EXAMPLE 6

This example describes the preparation of N-cyclohexyl-2-benzothiazole sulfenamide (CBS). Using the apparatus described in Example 1, 5.36 g of MBT (0.03 mole) was dissolved in a mixture of 89.2 g of cyclohexylamine (0.9 mole) and 10 g of water before 1.2 g of the activated carbon catalyst of Example 1 was added. The oxidation was carried out between 30-50°C at an oxygen gauge pressure of 0.12 MPa. The reaction was stopped after 1 hour when the ORP measurement reached a plateau. By HPLC analysis, the crude liquid consisted of 4.0% MBT, 92.8% CBS, 0.1% MBTS, and 2.0% of benzothiazole. The reaction was repeated using isopropanol as a solvent, with improved reaction rate and CBS yield.

### EXAMPLE 7

This example describes the preparation of N-isopropyl-2-benzothiazole sulfenamide. A mixture of 4.2 g of MBT (0.025 mole), 49.1 g of isopropylamine (0.83 mole), and 1.0 g of the activated carbon catalyst of Example 1 was placed in a 160 mL Parr autoclave equipped with a temperature control system, oxygen feed/reactor sampling system, and a stirrer. The autoclave was modified to permit the continued bleed-off of oxygen during the reaction while maintaining an autoclave pressure of 0.12 MPa. Oxygen feed was started at room temperature before the mixture was heated to 50°C. After a 2-hour reaction time at 50°C, the autoclave was allowed to cool to 30°C with slow release of pressure. The autoclave contents were purged with argon for about 3 min before the autoclave was opened. The catalyst was filtered off and rinsed with about 10 mL of isopropylamine to give a filtrate which was essentially a solution of N-isopropyl-2-benzothiazole sulfenamide in isopropylamine. The solution was quenched with 600 mL of water (6:1 v/v water:amine solution) to precipitate the sulfenamide. After stirring the slurry at room temperature for at least 15 min, the precipitate was filtered off, washed with 50 mL of water, and dried overnight at 50°C. The yield of N-isopropyl-2-benzothiazole sulfenamide was 94.6% (99.0% purity).

### EXAMPLE 8

This example describes the preparation of 2-(morpholinothio)benzothiazole. Using the apparatus and general procedure described in Example 7, the mixture of 4.2 g of MBT (0.025 mole), 72.3 g of morpholine (0.83 mole) and 1.0 g of the activated carbon catalyst of Example 1 was oxidized at 70°C under 0.12 MPa oxygen pressure for 90 min. After removal of the catalyst, the filtrate was quenched with water (10:1 v/v water:amine solution) to precipitate the sulfenamide. The yield of 2-(morpholinothio)benzothiazole was 71.4% (97.9% purity).

### EXAMPLE 9

This example describes the preparation of N-tert-butyl-2-thiazole sulfenamide. Using the apparatus and general procedure described in Example 7, the mixture of 4.7 g of 2-mercaptothiazole (0.040 mole), 60.8 g of tert-butylamine (0.93 mole), and 0.5 g of the activated carbon catalyst of Example 1 was oxidized at 60°C under 0.12 MPa pressure for 90 min. After removal of the catalyst, the filtrate was quenched with water (9:1 v/v water:amine solution) to precipitate the sulfenamide. The yield of N-tert-butyl-2-thiazole sulfenamide was 98.7% (97.9% purity).

### EXAMPLE 10

This example describes the preparation of N-tert-butyl-2-pyridine sulfenamide. The apparatus and general procedure described in Example 7 was used except that purge of oxygen was prevented during the reaction by completely sealing the autoclave. A mixture of 2.8 g of 2-mercaptopyridine (0.025 mole), 60.8 of tert-butylamine, and 1.0 g of the activated carbon catalyst of Example 1 was oxidized at 70°C under 0.34 MPa pressure for 7 hours. After this time, a sample of the liquid in the autoclave by HPLC analysis consisted of 88.0% N-tert-butyl-2-pyridine sulfenamide. The reaction was stopped, the catalyst filtered off, and the filtrate was quenched with water (10:1 v/v water:amine solution) to precipitate the sulfenamide. The yield of N-tert-butyl-2-pyridine sulfenamide was 60% (98.0% purity).

### EXAMPLE 11

This example describes the preparation of 2-(morpholinodithio)benzothiazole. A mixture of 25.0g MBT (0.150 mole), 13.6g of morpholine (0.156 mole), 4.8g of sulfur (0.150 mole), 157.0g of isopropanol, and 4.0g of the activated carbon catalyst of Example 1 was placed in a 300 mL Parr autoclave equipped with a temperature control system, oxygen feed/reactor sampling system, and a stirrer. The mixture was heated to 30°C and the oxygen pressure was set at 0.35 MPa and maintained at this pressure throughout the reaction. After 3 hours reaction time, oxygen uptake had essentially stopped.

The oxygen pressure was slowly released and the contents of the autoclave were heated to 75°C to ensure complete dissolution of the formed product. Oxygen pressure was reapplied, and this pressure was used to remove the isopropanol solution from the carbon catalyst. This solution was allowed to cool to room temperature and the product which precipitated out was filtered, washed with isopropanol and dried. The melting point of this product was 124-126°C. A second crop of product crystals was isolated by reducing the volume of mother liquor to approximately one-fifth its original volume and isolating the crystals. The final isolated product was 39.8g with purity of 93.5% as measured by HPLC. This chromatogram indicated no overoxidized products and showed only starting material along with the 2-(morpholinodithio)benzothiazole.

### EXAMPLE 12

This example describes the preparation of TBBS from bis(2,2'-benzothiazole) disulfide (MBTS). The apparatus and general procedure described in Example 7 was used except that purge of oxygen was prevented during the reaction by completely sealing the autoclave. A mixture of 13.3g of MBTS (0.04 mole), 60.8g of TBA (0.83 mole , 938% excess ) and 1.5g of the activated carbon catalyst of Example 1 was oxidized at 70°C under 0.343 MPa oxygen gauge pressure for two hours. After removal of the catalyst, the filtrate was quenched with water (10:1 v/v, water: amine solution) to precipitate TBBS in 95.8% yield (98.3% purity).

## Claims

1. A process for preparing sulfenamide by catalytic oxidation of a thiol of a nitrogen-containing heterocycle with oxygen in the presence of an amine selected from t-butylamine, cyclohexylamine, isopropylamine, morpholine and diisopropylamine, in which the catalyst is activated carbon and contains no metal complex of porphyrazine or a derivative of porphyrazine, which process is performed in a liquid reaction medium comprising protic or aprotic solvents and is performed at a temperature of from at least 25°C to 150 degrees C.

2. The process of Claim 1, in which the material to be oxidized is selected from 2-mercaptobenzothiazole, 2-pyridinethiol, 2-pyrimidinethiol, 4-pyrimidinethiol, 2-pyrazinethiol, 3-pyridazinethiol, 2-mercaptobenzimidazole, 2-quinolinethiol and 2-lepidinethiol.

3. The process of Claim 1, in which the activated carbon has been treated to remove oxides from its surface.

4. The process of Claim 2, in which the oxidation is performed in a reaction medium comprising an excess of the amine.

5. The process of Claim 1, in which the oxygen is mixed with an inert gas.

6. The process of Claim 1, in which oxygen is supplied at a gauge pressure of from 0.1 to 1.0 MPa.

7. The process of Claim 1, which is performed at a temperature of from 30° to 90°C.

8. The process of Claim 1, in which the carbon is present in an amount of from 1% to 75% by weight, based on the weight of the thiol or salt or dithiocarbamic acid or its salt.

## Patentansprüche

1. Verfahren zur Herstellung von Sulfenamid durch katalytische Oxidation eines Thiols eines Stickstoff enthaltenden Heterocyclus mit Sauerstoff in Gegenwart eines Amins, gewählt aus t-Butylamin, Cyclohexylamin, Isopropylamin, Morpholin und Diisopropylamin, wobei der Katalysator Aktivkohle ist und keinen Metallkomplex von Porphyrazin oder eines Derivates von Porphyrazin enthält, welches Verfahren in einem flüssigen Reaktionsmedium, umfassend protische oder aprotische Lösungsmittel, bei einer Temperatur von mindestens 25°C bis 150°C durchgeführt wird.

2. Verfahren nach Anspruch 1, wobei das zu oxidierende Material aus 2-Mercaptobenzothiazol, 2-Pyridinthiol, 2-Pyrimidinthiol, 4-Pyrimidinthiol, 2-Pyrazinthiol, 3-Pyridazinthiol, 2-Mercaptobenzimidazol, 2-Chinolinthiol und 2-Lepidinthiol gewählt wird.

3. Verfahren nach Anspruch 1, wobei die Aktivkohle behandelt worden ist, um Oxide von deren Oberfläche zu entfernen.

4. Verfahren nach Anspruch 2, wobei die Oxidation in einem Reaktionsmedium durchgeführt wird, das einen Überschuß des Amins umfaßt.

5. Verfahren nach Anspruch 1, wobei der Sauerstoff mit einem Inertgas vermischt wird.

6. Verfahren nach Anspruch 1, wobei Sauerstoff bei einem Überdruck von 0,1 bis 1,0 MPa zugeführt wird.

7. Verfahren nach Anspruch 1, welches bei einer Temperatur von 30 bis 90°C durchgeführt wird.

8. Verfahren nach Anspruch 1, wobei der Kohlenstoff in einer Menge von 1 bis 75 Gew. -% vorliegt, bezogen auf das Gewicht des Thiols oder Salzes oder Dithiocarbaminsäure oder deren Salz.

## Revendications

1. Procédé de préparation de sulfénamide par oxydation catalytique d'un thiol d'un composé hétérocyclique comportant un azote, avec l'oxygène en présence d'une amine sélectionnée parmi la t-butylamine, la cyclohexylamine, l'isopropylamine, la morpholine et la diisopropylamine, dans lequel le catalyseur est du charbon actif et ne contient pas de complexe de métal avec la porphyrazine ou un dérivé de la porphyrazine, ledit procédé étant mené dans un milieu réactionnel liquide comprenant des solvants protiques et aprotiques et étant mené à une température d'au moins 25°C à 150 °C.

2. Procédé selon la revendication 1 dans lequel la substance à oxyder est sélectionnée parmi le 2-mercaptobenzothiazole, le 2-pyridinethiol, le 2-pyrimidinethiol, le 4-pyrimidinethiol, le 2-pyrazinethiol, le 3-pyridazinethiol, le 2-mercaptobenzimidazole, le 2-quinolinethiol et le 2-lepidinethiol.

3. Procédé selon la revendication 1 dans lequel le charbon actif a été traité afin d'éliminer les oxydes de sa surface.

4. Procédé selon la revendication 2 dans lequel l'oxydation est effectuée dans un milieu réactionnel comprenant un excès d'amine.

5. Procédé selon la revendication 1 dans lequel l'oxygène est mélangé avec un gaz inerte.

6. Procédé selon la revendication 1 dans lequel est fourni à une pression au manomètre d'environ 0,1 à 1,0 MPa.

7. Procédé selon la revendication 1 mené une température de 30 à 90°C.

8. Procédé selon la revendication 1 dans lequel le charbon est présent en une quantité de 1 à 75 % en poids, basé sur le poids du thiol ou son sel ou l'acide dithiocarbamique ou son sel.
